(19) Europäisches Patentamt
European Patent Office
Office européen des brevets

(11) Publication number: **0 252 736 B1**

(12) **EUROPEAN PATENT SPECIFICATION**

(45) Date of publication of patent specification: **24.07.91**

(51) Int. Cl.$^5$: **C07C 67/36**, C07C 69/62

(21) Application number: **87306051.1**

(22) Date of filing: **08.07.87**

(54) **Process for preparing fluorine-containing carboxylic acid ester.**

(30) Priority: **11.07.86 JP 161915/86**
**19.12.86 JP 301332/86**

(43) Date of publication of application:
**13.01.88 Bulletin 88/02**

(45) Publication of the grant of the patent:
**24.07.91 Bulletin 91/30**

(84) Designated Contracting States:
**CH DE FR GB LI**

(56) References cited:

**DE-A- 2 137 712**

**TETRAHEDRON LETTERS, vol. 25, no. 3, 1984, pages 303-306, London, GB; T. FUCHIKAMI et al.: "Transition-metal complex catalyzed polyfluoroalkylation. I. Facile addition of polyfluoroalkyl halides to carbon-carbon multiple bonds"**

**THE JOURNAL OF THE ORGANIC CHEMISTRY, vol. 48, no. 21, 1983, pages 3803-3807, Columbus, Ohio, US; T. FUCHIKAMI et al.: "Regioselective hydroeterification and hydrocarboxylation of 3,3,3-trifluoropropene and pentafluorostyrene catalyzed by phosphine-palladium complex"**

(73) Proprietor: **SAGAMI CHEMICAL RESEARCH CENTER**
**4-5, Marunouchi 1-chome**
**Chiyoda-ku Tokyo 100(JP)**

(72) Inventor: **Fuchikami, Takamasa**
**No. 1-9-2, Minamidai**
**Sagamihara-shi Kanagawa(JP)**
Inventor: **Urata, Hisao**
**No. 3-16 Sakae-cho**
**Sagamihara-shi Kanagawa(JP)**
Inventor: **Ishii, Yoshimitsu**
**No. 2508, Sakai**
**Atsugi-shi Kanagawa(JP)**
Inventor: **Obata, Yoshiko**
**No. 2-10-21 Shimotakaido Suginami-ku**
**Tokyo(JP)**

(74) Representative: **Diamond, Bryan Clive et al**
**Gee & Co., Chancery House, Chancery Lane**
**London WC2A 1QU(GB)**

**EP 0 252 736 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to a process for preparing a fluorine-containing carboxylic acid ester. More specifically, this invention relates to a novel process for preparing a fluorine-containing carboxylic acid ester of a general formula shown below.

At present, fluorine-containing carboxylic acids are used for a wide variety of utilities such as surface active agents or surface treating agents because of their excellent stability, chemical resistance, weather resistance, water- and oil-repellency, and are also useful as intermediates for the synthesis of physiologically active substances. The fluorine-containing carboxylic acid esters obtained by the process of this invention are important intermediates for the synthesis of various useful compounds having the above-described properties.

Typical conventional processes for preparing fluorine-containing carboxylic acid ester derivatives comprise the Reppe method using a fluorine-containing substituted ethylene. Examples of the conventional processes include (l) a process comprising reacting a perfluorooctylethylene with carbon monoxide at 380 atms. in ethanol in the presence of a palladium catalyst and an alcoholic hydrogen chloride at a temperature of l40 °C for 7 hours to obtain a mixture of ethyl α- and β-perfluorooctylpropionate, as described in German OLS No. 2l377l2; (2) a process comprising reacting trifluoropropene with an alcohol in the presence of a divalent palladium catalyst having a tertiary phosphine ligand under a carbon monoxide pressure of ll0 atms. at a temperature of from l00 to l25 °C for a period of from 30 to 70 hours to obtain a mixture of ethyl α- and β-trifluoromethylpropionate, as described in T. Fuchikami, K. Ohishi and I. Ojima, J. Org. Chem., Vol. 48, 3803 (l983), etc. However, each of the above conventional processes (l) and (2) is not advantageous in that the selectivity to the position of carbon atom where carbonylation occurs is low. That is, although ethyl β-perfluoroalkyl propionate is produced preferentially, an α-substituted product is also produced in a substantial amount, and these products are difficult to separate into the respective pure product. Further, the above conventional processes require a carbon monoxide pressure higher than l00 atms. and, hence, are very expensive in order to ensure the safety of the process.

As a result of extensive studies for overcoming the disadvantages of the conventional processes, the present inventors found that a single carbonylated product, i.e., a fluorine-containing carboxylic acid ester represented by the formula (I) can be produced in high yield according to the process of this invention.

This invention relates to a process for preparing a fluorine-containing carboxylic acid ester represented by the formula (I)

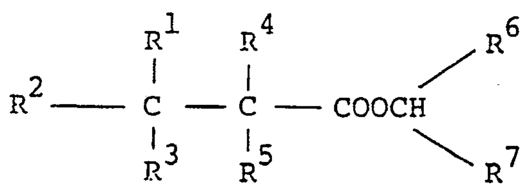

(I)

wherein $R^1$ represents a fluorine atom or a polyfluorocarbon group, $R^2$ and $R^3$ each represents a hydrogen atom, a fluorine atom, an alkyl group, an aralkyl group, an alkenyl group or an aryl group, $R^4$, $R^5$, $R^6$ and $R^7$ each represents a hydrogen atom, an alkyl group, an aralkyl group, an alkenyl group or an aryl group, and $R^2$ and $R^3$, $R^2$ and $R^4$, $R^3$ and $R^5$, $R^4$ and $R^5$, or $R^6$ and $R^7$, when taken together with the carbon atom(s) to which they are attached, can form a ring, which comprises reacting a fluorine-containing alkyl halide represented by the formula (II)

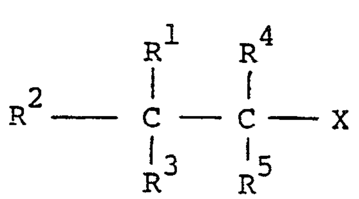

(II)

wherein $R^1$, $R^2$, $R^3$, $R^4$ and $R^5$ are as defined above, and X represents an iodine atom, a bromine atom or a

chlorine atom, with carbon monoxide and an alcohol represented by the formula (III)

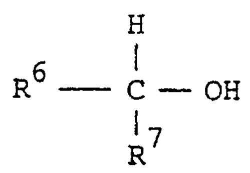 (III)

wherein $R^6$ and $R^7$ are as defined above, in the presence of a transition metal catalyst of the Group VIII of the Periodic Table and a base.

The term "alkyl group" as used herein means a straight or branched chain alkyl group having l to l0 carbon atoms or a cycloalkyl group having 3 to l0 carbon atoms.

The term "alkenyl group" as used herein means an alkenyl group having 2 to 10 carbon atoms.

The term "aryl group" as used herein means a phenyl or naphthyl group, or a phenyl group substituted with at least one of halogen atoms, alkyl, alkoxy, hydroxy, nitro, acyl, acyloxy and amino groups.

The term "aralkyl group" as used herein means an aralkyl group having the above aryl moiety and an alkyl group having l to l0 carbon atoms.

In the process of this invention, it is essential to conduct the reaction in the presence of a transition metal catalyst of the Group VIII of the Periodic Table. The transition metal catalysts which can be used include metallic iron, ruthenium, osmium, cobalt, rhodium, iridium, nickel, palladium or platinum; a metal salt, a metal complex compound, an organometallic complex containing a halogen atom ligand, an organometallic complex containing a tertially phosphine ligand, and an organometallic complex containing an olefin or acetylene compound as a ligand of the above metal, as well as the above transition metal or metal compound of the Group VIII supported on a carrier such as silica gel or alumina.

Suitable examples of the catalyst include iron carbonyl, ruthenium carbonyl, osmium carbonyl, nickel carbonyl, iron chloride, cobalt chloride, ruthenium chloride, rhodium chloride, nickel chloride, palladium chloride, platinum chloride, dichlorobis(triphenylphosphine)nickel, dichlorobis(triphenylphosphine)palladium, dichloro(l,2-bisdiphenylphosphinoethane)palladium, dichloro(l,3-bisdiphenylphosphinopropane)palladium, dichloro(l,4-bisdiphenylphosphinobutane)palladium, dichloro(l,l'-bisdiphenylphosphinoferrocene)palladium, dichlorobis(diphenylmethylphosphine)palladium, dichlorobis(triphenylphosphine)palladium, bis-(cyclooctadiene)nickel, dichloro(cyclooctadiene)palladium, tetrakis(triphenylphosphine)nickel, chlorotris-(triphenylphosphine)rhodium, chlorotris(triphenylphosphine)iridium, chlorocarbonylbis(triphenylphosphine)-rhodium, chlorocarbonylbis(triphenylphosphine)iridium and tetrakis(triphenylphosphine)palladium, tetrakis-(triphenylphosphine)platinum.

The transition metal catalyst of the Group VIII can be used in an amount of from 1/10000 to 1/5 molar equivalent to the fluorine-containing alkyl halide of the formula (II), preferably, l/500 to l/5 molar equivalent to the compound of formula (II).

The process according to the present invention is carried out in the presence of a base. Examples of bases which can be used in the present invention include inorganic bases such as hydrides, hydroxides, carbonates, bicarbonates or carboxylates of alkali metals or alkaline earth metals; alkali metal alkoxides and alkali metal amide; tertiary amines such as triethylamine, tri(isobutyl)amine or N,N-dimethylaniline; and aromatic amines such as pyridine or 2,6-lutidine. The base can be used in an amount of from 0.5 to 5 molar equivalent to the fluorine-containing alkyl halide of the formula (II).

The fluorine-containing alkyl halides represented by the formula (II) used as a starting material of this invention are easily available as commercial products, and examples of the alkyl halides include l,l,l-trifluoro-2-iodoethane, l,l,l-trifluoro-2-bromoethane, l,l,l-trifluoro-2-chloroethane, l,l,l-trifluoro-3-iodopropane, l,l,l-trifluoro-3-bromopropane, l,l,l-trifluoro-3-chloropropane, l-fluoro-2-iodopropane, l-perfluoroethyl-2-iodoethane, l-perfluoroethyl-2-bromoethane, l-perfluoroethyl-2-chloroethane, l-perfluoropropyl-2-iodoethane, l-perfluoroisopropyl-2-iodoethane, l-perfluorobutyl-2-iodoethane, l-perfluoropentyl-2-iodoethane, l-perfluorohexyl-2-iodoethane, l-perfluoroheptyl-2-iodoethane, l-perfluorooctyl-2-iodoethane, l-perfluorodecyl-2-iodoethane, l-perfluorocyclohexyl-2-iodoethane, l-perfluoropropyl-2-bromoethane, l-perfluoroisopropyl-2-bromoethane, l-perfluorobutyl-2-bromoethane, l-perfluoropentyl-2-bromoethane, l-perfluorohexyl-2-bromoethane, l-perfluoroheptyl-2-bromoethane, l-perfluorooctyl-2-bromoethane, l-perfluorodecyl-2-bromoethane, l-trifluoromethyl-2-iodopropane, l-perfluoroethyl-2-iodopropane, l-perfluoropropyl-2-iodopropane, l-perfluorobutyl-2-iodopropane, l-perfluorohexyl-2-iodopropane, l-perfluoroheptyl-2-iodopropane, l-perfluorooctyl-2-iodopropane, l-perfluorodecyl-2-iodopropane, l-trifluoromethyl-2-iodobutane,

l-perfluoroethyl-2-iodobutane, l-perfluoropropyl-2-iodobutane, l-perfluorobutyl-2-iodobutane, l-perfluorohexyl-2-iodobutane, l-perfluorooctyl-2-iodobutane, l-trifluoromethyl-2-iodopentane, l-perfluoroethyl-2-iodopentane, l-perfluoropropyl-2-iodopentane, l-perfluorobutyl-2-iodopentane, l-perfluorohexyl-2-iodopentane, l-perfluorooctyl-2-iodopentane, l-perfluoroethyl-2-iodohexane, l-perfluorobutyl-2-iodohexane, l-perfluorohexyl-2-iodohexane, l-perfluorooctyl-2-iodohexane, l-perfluorooctyl-2-iodooctane, l-perfluorohexyl-2-iodooctane, l-perfluorooctyl-2-iodocyclohexane, l-perfluorohexyl-2-iodocyclopentane, 5-iodo-6-perfluorooctyl-2-hexanone, l-perfluorobutyl-2-iodo-3-phenylpropane, l-perfluorohexyl-2-iodo-7-octene, l-perfluoroheptyl-2-bromo-9-decene, l-perfluorononyl-2-chloro-5-hexene, l-(3-cyclohexenyl)-l-iodoperfluorobutylethane, l-perfluorohexylmethyl-l-iodocyclohexane, l-(2,2,2-trifluoroethyl)-l-bromocyclohexane, l-perfluorooctylmethyl-l-chlorocyclopentane, l-perfluorobutylmethyl-l-iodo-4-cyclooctene, l-iodomethyl-l-perfluoroethylcyclohexane, l-bromomethyl-l-perfluorodecylcyclooctane, l-chloromethyl-l-trifluoromethylcyclopentane, l-perfluorooctyl-2-bromo-3-phenylpropane, l-iodo-l-trifluoromethyl-4-phenyl-3-propene, l-perfluorobutyl-2-iodo-4-methylhexane, l-perfluorohexyl-2-iodo-4-methylhexane, l-perfluorohexyl-2-iodo-4-methylhexane, l-perfluoropropyl-2-ethyl-2-iodohexane, l-perfluoroisopropyl-2-iodo-2,4-dimethylhexane, l-perfluorobutyl-2-iodo-2-cyclohexylethane, l-trifluoromethyl-2-iodo-2-cyclohexylethane, l-perfluoroethyl-2-iodo-2-cyclohexylethane, l-perfluorooctyl-2-iodo-2-cyclohexylethane, l-trifluoromethyl-2-iodo-2-cyclopentylethane, l-perfluoropropyl-2-iodo-2-cyclopentylethane and l-perfluorohexyl-2-iodo-2-cyclopentylethane.

The alcohols represented by the formula (III) which can be used in the present invention include straight or branched chain or cyclic alcohols such as methanol, ethanol, propanols, butanols, pentanols and hexanols; benzyl alcohol, 2-phenyl-1-ethanol, furfuryl alcohol and 3-butene-1-ol.

The alcohol is preferably used in an amount of at least an equimolar amount to the fluorine-containing alkyl halide represented by the formula (II), and any excess amount of the alcohol can be used so as to serve as a diluent.

The process according to the present invention is carried out in an atmosphere of carbon monoxide which may be diluted with an inert gas such as argon, nitrogen or the like.

The reaction proceeds efficiently under a carbon monoxide partial pressure below about 50 atms., but a higher pressure can be used, if desired.

In carrying out the process of this invention, an inert solvent which does not take part in the reaction may be additionally used, if desired. A preferred solvent includes that forms a single reaction phase, but the solvent which forms a second liquid phase in the reaction mixture may also be used. Examples of solvents which can be preferably used include hydrocarbon solvents such as hexane, heptane, cyclohexane, benzene, toluene, xylene, etc., and polar solvents such as acetonitrile, dichloromethane, acetone, chloroform, diethyl ether, tetrahydrofuran, dioxane, etc.

The reaction according to the present invention can be carried out at a temperature of from about 20 to about l50° C, preferably 40 to l20° C, for a period of from about 2 to about 50 hours, preferably from 8 to 40 hours.

The reaction of the present invention can be conducted in a pressure-resistant sealed vessel, e.g., in an autoclave, by charging the starting materials, the catalyst, the base and the solvent, if used, and then charging carbon monoxide to a predetermined pressure, and, after sealing the reaction vessel, heating the mixture, preferably with stirring.

Alternatively, in the process of this invention, the fluorine-containing alkyl halide represented by the formula (II) wherein $R^1$ represents a polyfluorocarbon group, one of $R^2$ and $R^3$ represents a hydrogen atom and the other of $R^1$ and $R^3$ represents a hydrogen atom, an alkyl group, or an aralkyl group can be formed in the reaction system <u>in situ</u> by using an olefin of the formula (IV)

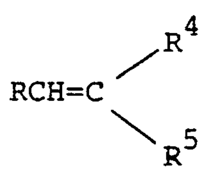

(IV)

wherein R represents a hydrogen atom, an alkyl group or an aralkyl group, and a polyfluoroalkyl halide of the formula (V)

$R^1X$ (V)

wherein $R^1$ represents a polyfluorocarbon group and X represents an iodine atom, a bromine atom or a chlorine atom.

As described in T. Fuchikami and I. Ojima, Tetrahedron Lett., Vol. 25, 303, 307 (l984), the above polyfluoroalkyl halide of the formula (V), in particular, that having a perfluoroalkyl group for $R^1$ and an iodine atom for X, easily undergoes an addition reaction to the above olefin of the formula (IV) in the presence of a catalyst of transition metal complex (e.g., a complex of Fe, Co, Ru, Pd, Ni, etc.) to form a compound represented by the formula (VI)

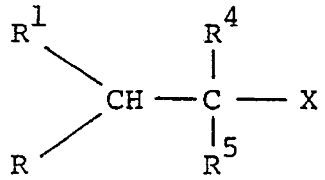

(VI)

wherein R, $R^1$, $R^4$ and $R^5$ are as defined above, which corresponds to a starting material represented by the formula (II). Thus, the starting material of the formula (II) can be formed in situ in the reaction system from the compounds of formulae (IV) and (V).

The present invention is further illustrated in greater detail by the following Examples, but is not limited thereto. Unless otherwise indicated, all percents, parts, ratios and the like are by weight. The symbols "Me", "Et", "Bu" and "Ph" stand for a methyl group, an ethyl group, a butyl group and a phenyl group, respectively.

Example 1

$C_8F_{17}CH_2CH_2I$ + CO + EtOH → $C_8F_{17}CH_2CH_2COOEt$

A 30 ml stainless steel autoclave was charged with dichlorobis(triphenylphospine)palladium $[(Ph_3P)_2PdCl_2]$ (l4.4 mg, 0.02 mmol), l-perfluorooctyl-2-iodoethane (0.288 g, 0.50 mmol) and $Et_3N$ (70 $\mu$l, 0.50 mmol), and, after carbon monoxide was charged into the autoclave at 30 atms., the mixture was reacted at 80 °C for 40 hours. The reaction mixture was extracted with hexane, and the extract was washed with water and dried over magnesium sulfate. The solvent was distilled off, and the resulting residue was subjected to isolation and purification by silica gel column chromatography to recover l4% l-perfluoro-2-iodoethane and to obtain ethyl 3-perfluorooctylpropionate in a yield of 74% (86% conversion yield).

IR (neat)$\nu_{C=O}$ : l745 $cm^{-1}$

$^1$H-NHR ($CDCl_3$, TMS)$\delta$: l.28 (3H, t, J = 7Hz), 2.2 -2.8 (4H, br), 4.l8 (2H, q, J = 7Hz).

Example 2

$C_8F_{17}CH_2CH_2I$ + CO + EtOH → $C_8F_{17}CH_2CH_2COOEt$

A 20 ml stainless steel autoclave was charged with $C_8F_{17}CH_2CH_2I$ (0.574 g, l mmol), $Co_2(CO)_8$ (34 mg, 0.l mmol), EtOH (3 ml) and $Et_3N$ (0.l27 ml, 0.9 mmol), and, after carbon monoxide was charged into the autoclave at 50 atms., the mixture was stirred at l00 °C for 24 hours. The reaction mixture was extracted with diethyl ether, washed with water and dried over magnesium sulfate. The solvent was distilled off under reduced pressure, and the resulting residue was isolated and purified by silica gel column chromatography to obtain 0.286 g (55% yield) of ethyl 3-perfluorooctylpropionate.

Example 3

$C_6F_{13}CH_2CH_2I$ + CO + (i)BuOH →
$C_6F_{13}CH_2CH_2COO(i)Bu$

A 30 ml stainless steel autoclave was charged with $C_6F_{13}CH_2CH_2I$ (0.268 ml, l mmol), $(Ph_3P)_2PdCl_2$ - (35.5 mg, 0.05 mmol), $Et_3N$ (0.l4 ml, l mmol) and (i)BuOH (2 ml), and, after carbon monoxide was charged into the autoclave at 30 atms., the mixture was reacted at l00° C for 24 hours. The reaction mixture was isolated and purified by silica gel column chromatography to obtain 0.l9 g (42% yield) of isobutyl 3-perfluorohexylpropionate.
IR (neat) $\nu_{C=O}$ : l735 $cm^{-1}$.
$^1$H-NMR ($CDCl_3$, TMS) $\delta$: 0.93 (6H, d, J=7Hz), l.97
(lH, sep, J=7Hz), 2.23 - 2.9 (4H, br), 3.94 (2H, d, J=7Hz)

Example 4

$C_6F_{13}CH_2CH_2I$ + CO + EtOH →
$C_6F_{13}CH_2CH_2COOEt$

The reaction was conducted in the same manner as described in Example 3, except that EtOH (2 ml) was used instead of (i)BuOH used in Example 3, and 0.24 g (57% yield) of ethyl 3-perfluorohexylpropionate was obtained.
IR (neat) $\nu_{C=O}$ : l740 $cm^{-1}$.
$^1$H-NMR ($CDCl_3$, TMS) $\delta$: l.27 (3H, t, J=7Hz), 2.l -3.0 (4H, br), 4.2 (2H, q, J=7Hz).

Example 5

$C_4H_9CH_2CH_2I$ + CO + (i)BuOH →
$C_4H_9CH_2CH_2COO(i)Bu$

The reaction was conducted in the same manner as described in Example 3, except that $C_4H_9CH_2CH_2I$ (0.l92 ml, l mmol) was used instead of $C_6F_{13}CH_2CH_2I$ used in Example 3, and 0.l63 g (47% yield) of isobutyl 3-perfluorobutylpropionate was obtained.
IR (neat) $\nu_{C=O}$ : l740 $cm^{-1}$.
$^1$H-NMR ($CDCl_3$, TMS) $\delta$: 0.93 (6H, d, J=7Hz), l.97
(lH, sept, J=7Hz), 2.22 - 3.0 (4H, br), 3.93 (2H, d, J=7Hz).

Example 6

$C_4F_9CH_2CH_2I$ + CO + (n)BuOH →
$C_4F_9CH_2CH_2COO(n)Bu$

The reaction was conducted in the same manner as described in Example 5, except that (n)BuOH (0.9l ml, 9.9 mmol) and heptane (0.09 ml) were used instead of (i)BuOH used in Example 5, and 0.l5 g (44% yield) of butyl 3-perfluorobutylpropionate was obtained.
IR (neat) $\nu_{C=O}$ : l740 $cm^{-1}$.
$^1$H-NMR ($CDCl_3$, TMS) $\delta$ : 0.95 (3H, t, J=7.2Hz),
l.l3 - 2.0 (4H, m), 2.l - 2.9 (4H, br), 4.l7 (2H, t, J=7Hz).

Example 7

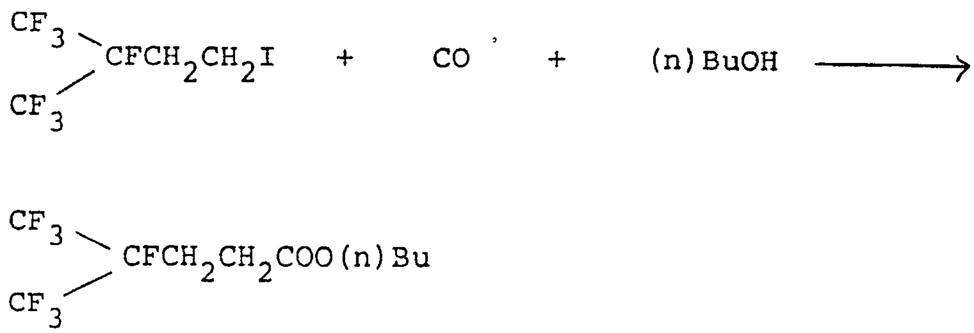

The reaction was conducted in the same manner as described in Example 3, except that $(CF_3)_2CFCH_2CH_2I$ (0.168 ml, 1 mmol) was used instead of $C_6F_{13}CH_2CH_2I$ and (n)BuOH (2 ml) was used instead of (i)BuOH used in Example 3, and 0.104 g (35% yield) of butyl 3-perfluoroisopropylpropionate was obtained.
IR (neat) $\nu_{C=O}$ 1740 $cm^{-1}$.
$^1$H-NMR ($CDCl_3$, TMS) $\delta$ : 0.94 (3H, t, J = 7.2Hz),
1.1 - 1.9 (4H, br), 2.2 - 2.9 (4H, br), 4.15 (2H, t, J = 7.2Hz).

Example 8

$CF_3CH_2CH_2I$ + CO + EtOH →
$CF_3CH_2CH_2COOEt$

A 20 ml stainless steel autoclave was charged with $(Ph_3P)_2PdCl_2$ (34.7 mg, 0.05 mmol), $CF_3CH_2CH_2I$ (56 µl, 0.5 mmol), $Et_3N$ (70 µl, 0.50 mmol) and EtOH (1 ml), and, after carbon monoxide was charged into the autoclave at 50 atms., the mixture was stirred at 120° C for 24 hours. The reaction mixture was quantitatively analyzed by gas chromatography and found that ethyl 4,4,4-trifluorobutylate was produced in 93% yield.

Example 9

$CF_3CH_2CH_2I$ + CO + EtOH →
$CF_3CH_2CH_2COOEt$

The reaction was conducted in the same manner as described in Example 8, except that $Co_2(CO)_8$ (17.1 mg, 0.05 mmol) was used instead of $(Ph_3P)_2PdCl_2$ and the reaction temperature of 120° C was used instead of 100° C used in Example 8, and ethyl 4,4,4-trifluorobutylate was obtained in a yield of 65%.

Example 10

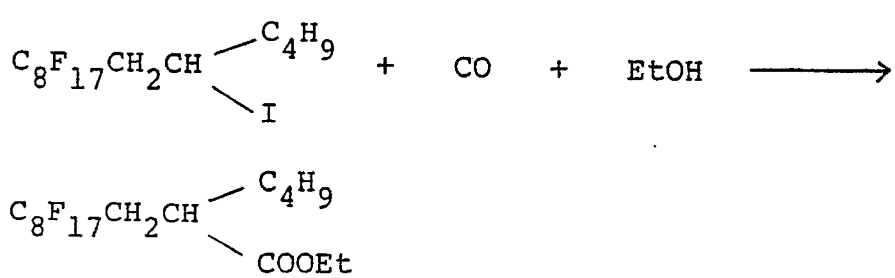

A 30 ml of stainless steel autoclave was charged with $(Ph_3P)_2PdCl_2$ (34.8 mg, 0.05 mmol), $C_8F_{17}CH_2CH(C_4H_9)I$ (360 µl, 1 mmol), $Et_3N$ (0.14 ml, 1.0 mmol) and EtOH (1 ml), and, after carbon monoxide was charged into the autoclave at 30 atms, the mixture was stirred at 80 °C for 12 hours. The reaction mixture was isolated and purified by silica gel column chromatography to obtain 0.43 g (75% yield) of ethyl 2-butyl-3-perfluorooctylpropionate.
$^1$H-NMR ($CDCl_3$, TMS) $\delta$ : 0.90 (3H, t, J=7Hz),
1.27 (3H, t, J=7Hz), 1.32 (4H, m), 1.58 (1H, m),
1.70 (1H, m), 2.12 (1H, m), 2.68 (1H, m), 2.78 (1H, m), 4.18 (3H, q, J=7Hz).

Example 11

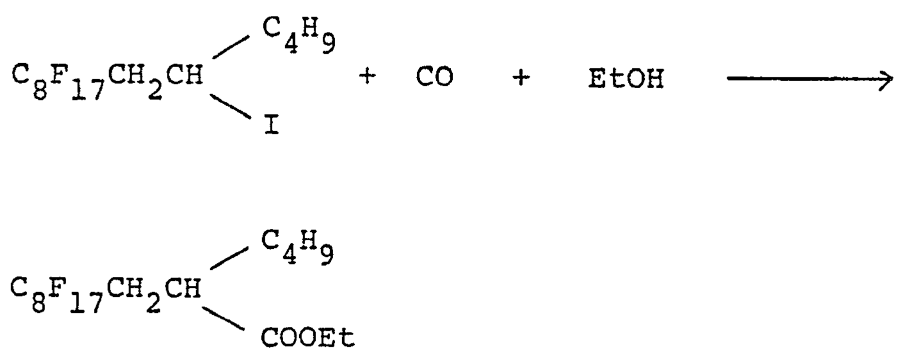

The reaction was conducted in the same manner as described in Example 10, except that a mixture of EtOH (0.6 ml) and heptane (0.4 ml) was used instead of EtOH (1 ml) used in Example 10, and 0.423 g (73% yield) of ethyl 2-butyl-3-perfluorooctylpropionate was obtained.

Example 12

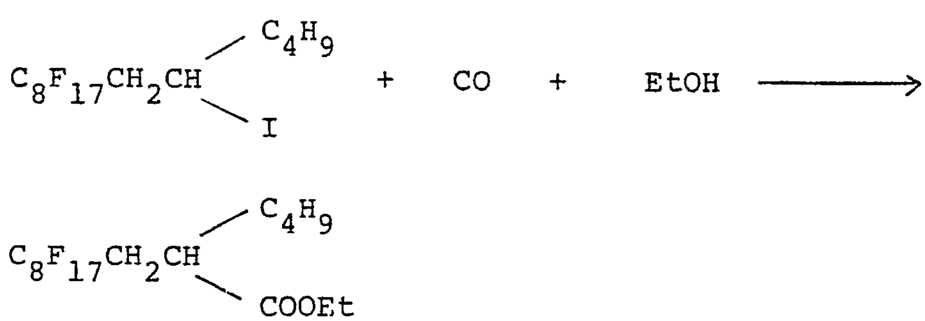

The reaction was conducted in the same manner as described in Example 11, except that dichloro(1,4-bisdiphenylphosphinobutane)palladium (30.4 mg, 0.05 mmol) was used instead of $(Ph_3P)_2PdCl_2$ used in Example 11, and 0.323 g (56% yield) of ethyl 2-butyl-3-perfluorooctylpropionate was obtained.

Example 13

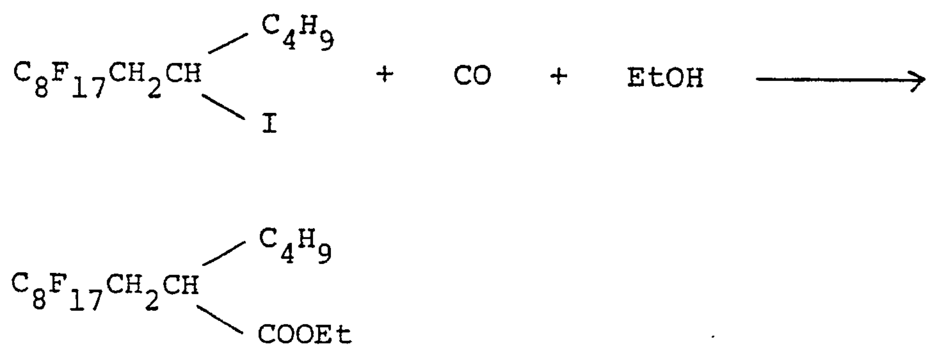

The reaction was conducted in the same manner as described in Example II, except that dichloro(I,2-bisdiphenylphosphinoethane)palladium (29.I mg, 0.05 mmol) was used instead of $(Ph_3P)_2PdCl_2$ used in Example II, and 0.326 g (57% yield) of ethyl 2-butyl-3-perfluorooctylpropionate was obtained.

Example I4

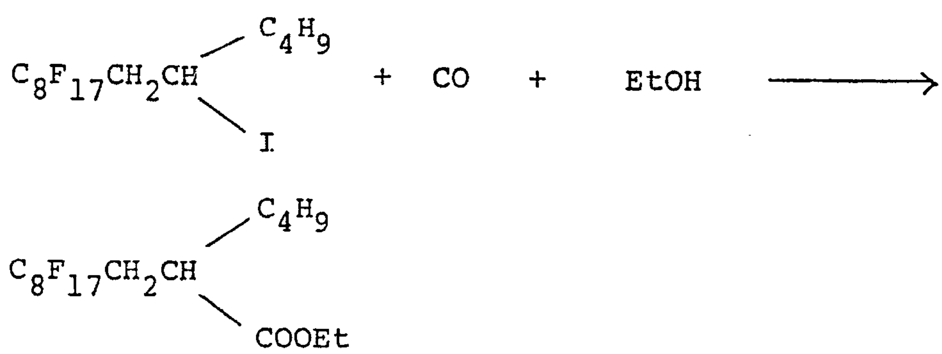

The reaction was conducted in the same manner as described in Example II, except that dichloro(I,I'-bisdiphenylphosphinoferrocene)palladium (36.7 mg, 0.05 mmol) was used instead of $(Ph_3P)_2PdCl_2$, and 0.476 (83% yield) of ethyl 2-butyl-3-perfluorooctylpropionate was obtained.

Example I5

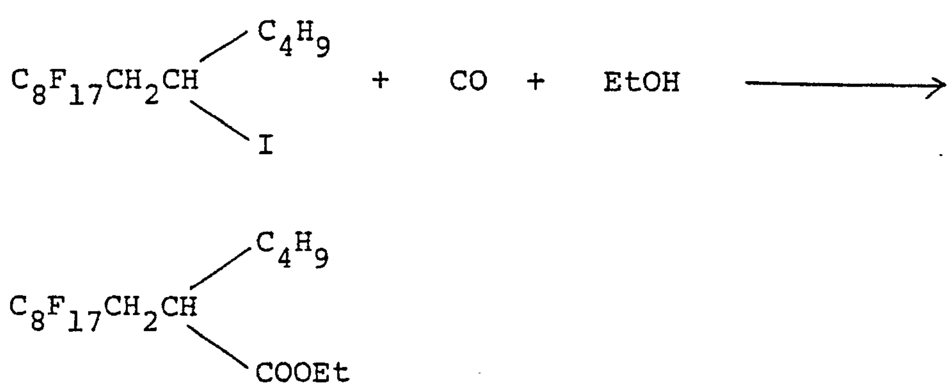

The reaction was conducted in the same manner as described in Example II, except for using dichloro-(I,3-bisdiphenylphosphinopropane)palladium (29.9 mg, 0.05 mmol) instead of $(Ph_3P)_2PdCl_2$ used in Example II, and 0.3I2 g (54% yield) of ethyl 2-butyl-3-perfluorooctylpropionate was obtained.

Example I6

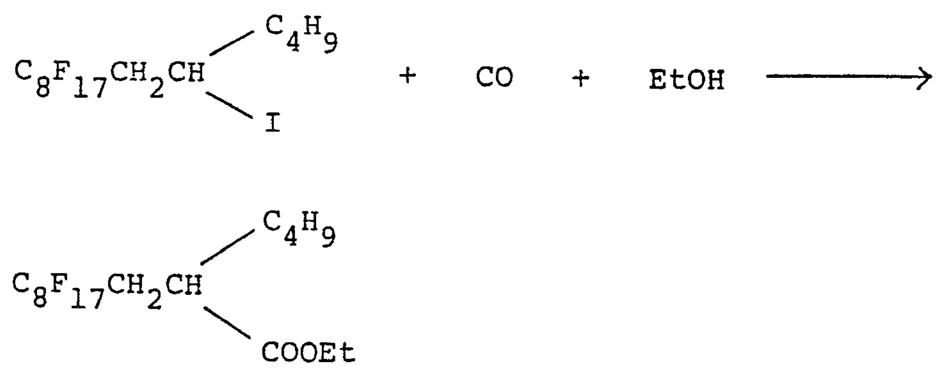

The reaction was conducted in the same manner as described in Example II, except that $RhCl(PPh_3)_3$ - (46.2 mg, 0.05 mmol) was used instead of $(Ph_3P)_2PdCl_2$ used in Example II, and 0.275 g (48% yield) of ethyl 2-butyl-perfluorooctylpropionate was obtained.

Example I7

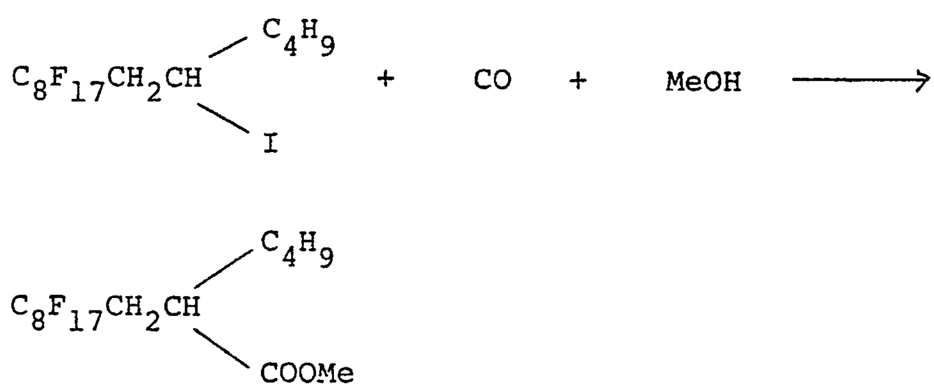

The reaction was conducted in the same manner as described in Example I0, except that a mixture of methanol (0.4 ml) and heptane (0.6 ml) was used instead of EtOH (I ml) used in Example I0, and 0.356 g (68% yield) of methyl 2-butyl-3-perfluorooctylpropionate was obtained.
IR (neat) $\nu_{C=O}$ : I745 $cm^{-1}$.
$^1$H-NMR ($CDCl_3$, TMS) $\delta$ : 0.9 (3H, t, J = 7Hz), I.I -I.9 (6H, m), I.93 - 3.0 (3H, m), 3.75 (3H, s).

Example I8

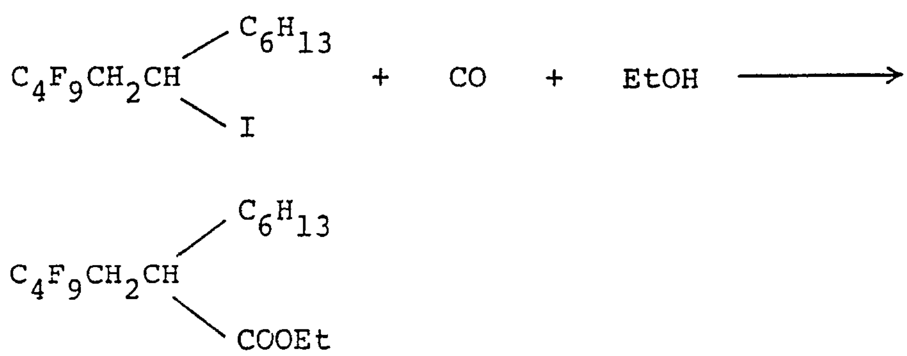

The reaction was conducted in the same manner as described in Example 10, except that $C_4F_9CH_2CH(C_6H_{13})I$ (0.30 ml, 1 mmol) was used instead of $C_8F_{17}CH_2CH(C_4H_9)I$ used in Example 10, and 0.22 g (54% yield) of ethyl 2-hexyl-3-perfluorobutylpropionate was obtained.
IR (neat) $\nu_{C=O}$ : 1735 $cm^{-1}$.
$^1$H-NMR ($CDCl_3$, TMS) $\delta$: 0.9 (3H, br), 1.27 (13H, br), 1.9 - 3.0 (3H, m), 4.22 (2H, q, J = 7.2Hz).

Example 19

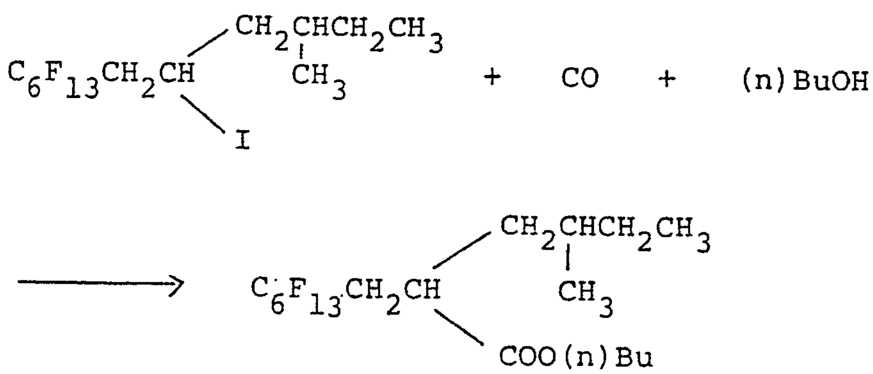

The reaction was conducted in the same manner as described in Example 18, except that $C_6F_{13}CH_2CH(I)CH_2CH(CH_3)CH_2CH_3$ (0.328 ml, 1 mmol) and (n)BuOH were used instead of $C_4F_9CH_2CH(C_6H_{13})I$ and EtOH used in Example 18, and 0.24 g (46% yield) of butyl 2-(2-methylbutyl)-3-perfluorohexylpropionate.
IR (neat) $\nu_{C=O}$ 1745 $cm^{-1}$.
$^1$H-NMR ($CDCl_3$, TMS) $\delta$ : 0.93 (9H, br), 1.1 - 2.1 (9H, br), 2.1 - 3.1 (3H, m), 4.13 (2H, t, J = 7Hz).

Example 20

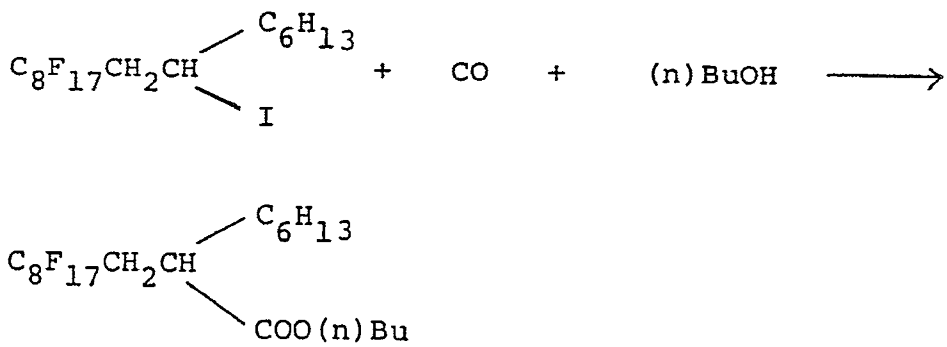

The reaction was conducted in the same manner as described in Example I9, except that $C_8F_{17}CH_2CH(C_6H_{13})I$ (0.394 ml, I mmol) was used instead of $C_6F_{13}CH_2CH(I)CH_2CH(CH_3)CH_2CH_3$ in Example I9, and 0.34 g (54% yield) of butyl 2-hexyl-3-perfluorooctylpropionate was obtained.
IR (neat) $\nu_{C=O}$ : I735 $cm^{-1}$.
$^1$H-NMR ($CDCl_3$, TMS) $\delta$ : 0.93 (6H, br), I.3 (I4H, br), I.9 - 3.0 (3H, m), 4.I4 (2H, t, J-_7Hz)

Example 2I

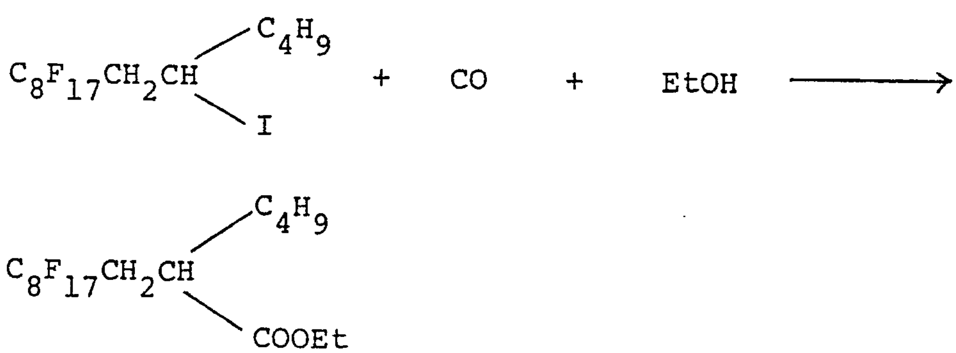

A 30 ml stainless steel autoclave was charged with $(Ph_3P)_2PdCl_2$ (4.I mg, 0.0065 mmol), potassium carbonate (36.I mg, 0.26 mmol), I-perfluorooctyl-2-iodohexane (40 µl) and ethanol (0.5 ml), and, after carbon monoxide was charged into the autoclave at I0 atms., the mixture was reacted at 80 °C for I2 hours to obtain ethyl 2-butyl-3-perfluorooctylpropionatein 47% yield.
$^1$H-NMR ($CDCl_3$, TMS) $\delta$: 0.90 (3H, t, J=7Hz), I.27 (3H, t, J=7Hz), I.32 (4H, m), I.58 (IH, m), I.70 (IH, m), 2.I2 (IH, m), 2.68 (IH, m), 2.78 (IH, m), 4.I8 (3H, q, J=7Hz).

Example 22

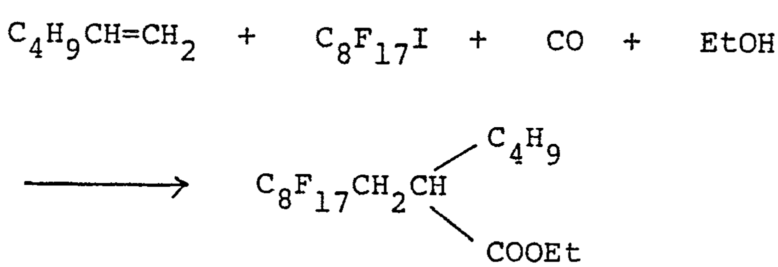

A 30 ml of stainless steel autoclave was charged with dichlorobis(triphenylphosphine)palladium (l2.6 mg, 0.0l7 mmol), potassium carbonate (73.2 mg, 0.52 mmol), ethanol (l ml), l-hexene (l08 µl, 0.86 mmol) and $C_8F_{17}I$(l32 µl, 0.50 mmol), and, after carbon monoxide was charged into the autoclave at 30 atms., the mixture was reacted at 80 °C for l2 hours. The reaction mixture was extracted with n-hexane, washed with water and dried over magnesium sulfate. The solvent was distilled off under reduced pressure. The quantitative analysis of the resulting residue showed that ethyl 2-butyl-3-perfluorooctylpropionate was produced in 67% yield.

Example 23

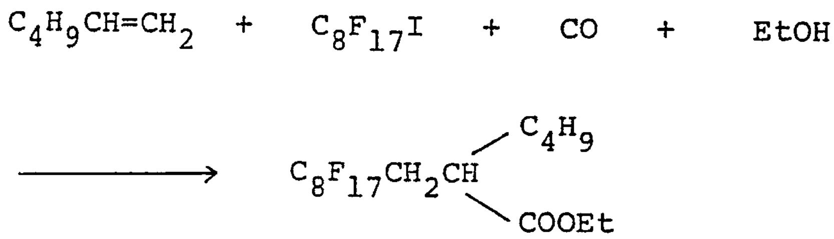

The reaction was conducted in the same manner as described in Example 22, except that dichlorobis-(triphenylphosphine)platinum was used instead oF dichlorobis(triphenylphosphine)palladium used in Example 22, and ethyl 2-butyl-3-perfluorooctylpropionate was obtained in 39% yield.

Example 24

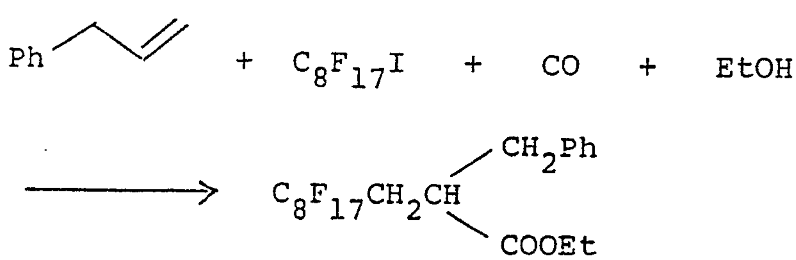

A stainless steel autoclave was charged with $(Ph_3P)_2PdCl_2$ (13.9 mg, 0.019 mmol), ethanol (1.5 ml), allylbenzene (131 )µl, l mmol), $C_8F_{17}I$ (132 µl, 0.50 mmol) and triethylamine (70 µl, 0.50 mmol), and carbon monoxide was charged into the autoclave at 30 atms. The mixture was then reacted at 80 °C for 24 hours to obtain ethyl 2-benzyl-3-perfluorooctylpropionate in 37% yield.
IR (neat) $\nu_{C=O}$ : 1742 $cm^{-1}$.
$^1$H-NMR (CDC1$_3$ TMS) δ: 1.16 (3H, t, J = 7Hz),
l.87 - 3.27 (5H, br), 4.l3 (2H, q, J = 7Hz), 7.3 (5H, m)
N.B 1 atm = 1.01325 bar.

## Claims

**1.** A process for preparing a fluorine-containing carboxylic acid ester represented by the formula (I)

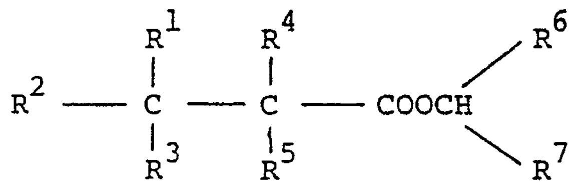

(I)

wherein $R^1$ represents a fluorine atom or a polyfluorocarbon group, $R^2$ and $R^3$ each represents a hydrogen atom, a fluorine atom, an alkyl group, an aralkyl group, an alkenyl group or an aryl group, $R^4$, $R^5$, $R^6$ and $R^7$ each represents a hydrogen atom, an alkyl group, an aralkyl group, an alkenyl group or an aryl group, and $R^2$ and $R^3$, $R^2$ and $R^4$, $R^3$ and $R^5$, $R^4$ and $R^5$ or $R^6$ and $R^7$, when taken together with the carbon atom(s) to which they are attached, can form a ring, and each alkyl or alkenyl moiety has not more than 10 carbon atoms and each aryl moiety is a phenyl or naphthyl moiety or phenyl substituted with at least one of halogen atoms and alkyl, alkoxy, hydroxy, nitro, acyl, acyloxy and amino groups; which comprises reacting a fluorine-containing alkyl halide represented by the formula (II)

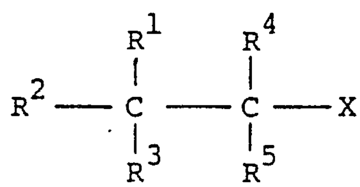

(II)

wherein $R^1$, $R^2$, $R^3$, $R^4$ and $R^5$ are as defined above, and X represents an iodine atom, a bromine atom or a chlorine atom, with carbon monoxide and an alcohol represented by the formula (III)

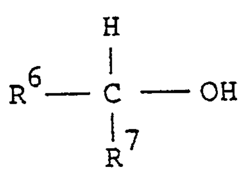

(III)

wherein $R^6$ and $R^7$ are as defined above, in the presence of a transition metal catalyst of the Group VIII of the Periodic Table and a base.

2. A process as claimed in Claim 1, wherein said reaction is conducted in a sealed vessel at a temperature of from 20 to 150 °C.

3. A process as claimed in Claim 1 or 2, wherein said transition metal catalyst is metallic iron, ruthenium, osmium, cobalt, rhodium, iridium, nickel, palladium or platinum, or a compound thereof.

4. A process as claimed in Claim 1, 2 or 3, wherein said transition metal catalyst is used in an amount of from 1/10000 to 1/5 molar equivalent to the fluorine-containing alkyl halide of the formula (II).

5. A process as claimed in any of Claims 1 to 4, wherein said base is used in an amount of from 0.5 to 5 molar equivalent to the fluorine-containing alkyl halide of the formula (II).

6. A process as claimed in any preceding claim, wherein said reaction is conducted in the presence of an inert solvent.

## Revendications

1. Procédé de préparation d'un ester d'acide carboxylique contenant du fluor représenté par la formule (I)

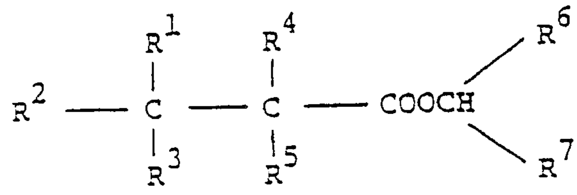

(I)

dans laquelle $R^1$ représente un atome de fluor ou un groupe polyfluorocarboné, $R^2$ et $R^3$ représentent chacun un atome d'hydrogène, un atome de fluor, un groupe alkyle, un groupe aralkyle, un groupe alkényle ou un groupe aryle, $R^4$, $R^5$, $R^6$ et $R^7$ représentent chacun un atome d'hydrogène, un groupe alkyle, un groupe aralkyle, un groupe alkényle ou un groupe aryle, et $R^2$ et $R^3$, $R^2$ et $R^4$, $R^3$ et $R^5$, $R^4$ et $R^5$ ou $R^6$ et $R^7$, lorsqu'ils sont pris ensemble avec l'(les) atome(s) de carbone auxquels ils sont liés, peuvent former un anneau, et chaque fraction alkyle ou alkényle n'a pas plus de 10 atomes de carbone et chaque fraction aryle est une fraction phényle ou naphthyle, ou phényle substitué avec au moins un des atomes d'halogène et des groupes alkyle, alkoxy, hydroxy, nitro, acyle, acyloxy et amino; procédé comprenant la réaction d'un halogénure d'alkyle contenant du fluore représenté par la formule (II)

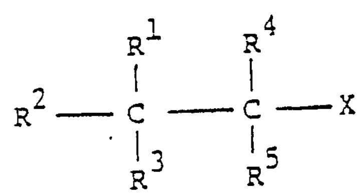

(II)

dans laquelle $R^1$, $R^2$, $R^2$, $R^4$ et $R^5$ sont tels que définis ci-dessus, et X représente un atome de iode, un atome de brome ou un atome de chlore, avec du monoxyde de carbone et un alcool représenté par la formule

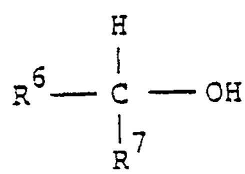

(III)

dans laquelle $R^6$ et $R^7$ sont tels que définis ci-dessus, en présence d'un catalyseur de métal de transition du Groupe VIII du Tableau périodique des éléments et d'une base.

2. Procédé selon la revendication 1, dans lequel on conduit la réaction dans un récipient scellé à une température allant de 20 à 150 °C.

3. Procédé selon la revendication 1 ou la revendication 2, dans lequel le dit catalyseur de métal de transition est le métal de fer, de ruthénium, osmium, cobalt, rhodium, iridium, nickel, palladium ou platine, ou un composé de ceux-ci.

4. Procédé selon la revendication 1, 2 ou 3, dans lequel on utilise ledit catalyseur de métal de transition dans une quantité allant de 1/10000 à 1/5 équivalente molaire à l'halogénure d'alkyle contenant du fluor de la formule (II).

5. Procédé selon l'une quelconque des revendications 1 à 4, dans lequel on utilise la dite base dans une quantité allant de 0,5 à 5 équivalente molaire à l'halogénure d'alkyle contenant du fluor de la formule (II).

6. Procédé selon l'une quelconque des revendications précédentes, dans lequel on conduit la réaction en présence d'un solvant inerte.

**Patentansprüche**

1. Verfahren zur Herstellung von fluorhaltigen Carbonsäureestern der Formel I

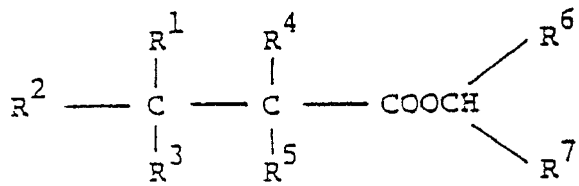

(I)

worin $R^1$ ein Fluoratom oder eine Polyfluorkohlenstoffgruppe bedeutet, $R^2$ und $R^3$ je ein Wasserstoffatom, ein Fluoratom, eine Alkylgruppe, eine Aralkylgruppe, eine Alkenylgruppe oder eine Aralkylgruppe darstellen, $R^4$, $R^5$, $R^6$ und $R^7$ je ein Wasserstoffatom, eine Alkylgruppe, eine Aralkylgruppe, eine Alkenylgruppe oder eine Arylgruppe darstellen und $R^2$ und $R^3$, $R^2$ und $R^4$, $R^3$ und $R^5$, $R^4$ und $R^5$ oder $R^6$ und $R^7$ zusammen mit dem (den) Kohlenstoffatom(en), an welche(s) sie gebunden sind, einen Ring bilden können und jeder Alkyl- oder Alkenylrest nicht mehr als 10 Kohlenstoffatome besitzt und jeder Arylrest ein Phenyl- oder Naphthylrest oder Phenyl ist, das mindestens mit einem der Halogenatome und Alkyl-, Alkoxy-, Hydroxy-, Nitro-, Acyl-, Acyloxy- und Aminogruppen substituiert ist, dadurch gekennzeichnet, dass ein fluorhaltiges Alkylhalogenid der Formel II

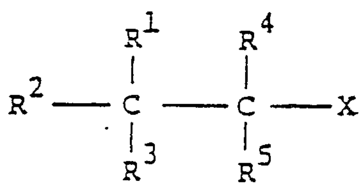

(II)

worin $R^1$, $R^2$, $R^3$, $R^4$ und $R^5$, wie oben definiert sind, und X ein Iodatom, ein Bromatom oder ein Chloratom bedeutet, mit Kohlenmonoxid und einem Alkohol der Formel III

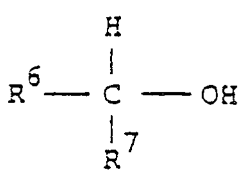

(III)

worin $R^6$ und $R^7$, wie oben definiert, sind, in Gegenwart eines Uebergangs-Metallkatalysators der Gruppe VIII des Periodensystems und einer Base umgesetzt wird.

2. Verfahren gemäss Anspruch 1, worin die genannte Reaktion in einem geschlossenen Gefäss bei einer Temperatur von 20 bis 150 °C umgesetzt wird.

3. Verfahren gemäss Anspruch 1 oder 2, worin der genannte Uebergangs-Metallkatalysator metallisches Eisen, Ruthenium, Osmium, Kobalt, Rhodium, Iridium, Nickel, Palladium oder Platin oder eine Verbindung davon ist.

4. Verfahren gemäss Anspruch 1, 2 oder 3, worin der genannte Uebergangs-Metallkatalysator in einer Menge von 1/10'000 bis 1/5 Molequivalent zum fluorhaltigen Alkylhalogenid der Formel II verwendet

wird.

5. Verfahren gemäss einem der Ansprüche 1 bis 4, worin die genannte Base in einer Menge von 0,5 bis 5 Molequivalente zum fluorhaltigen Alkylhalogenid der Formel II verwendet wird.

6. Verfahren gemäss einem der vorhergehenden Ansprüche, worin die Reaktion in Gegenwart eines inerten Lösungsmittels durchgeführt wird.